# NEW EUROPEAN PATENT SPECIFICATION

(11) **EP 1 152 787 B2**
(45) Date of publication and mention of the opposition decision: **04.09.2019**
(45) Mention of the grant of the patent: 10.08.2016
(21) Application number: 99969211.4
(22) Date of filing: 23.12.1999
(51) Int. Cl.: A61M 16/20, A61M 16/08, A61M 16/06

(54) **AN ANTI-ASPHYXIA VALVE**
ANTI-ERSTICKUNGSVENTIL
VALVE ANTI-ASPHYXIE

(30) Priority: 24.12.1998 AU PP794598
(43) Date of publication of application: 14.11.2001
(73) Proprietor: ResMed Pty Ltd, Bella Vista, NSW 2153 (AU)
(72) Inventor: WALKER, Adrienne, Mary, Claire, Summer Hill, NSW 2113 (AU); CRUMBLIN, Geoffrey, Baulkham Hills, NSW 2153 (AU); SMART, Gregory, Scott, Randwick, NSW 2031 (AU); GUNARATNAM, Michael, Kassipillai, Marsfield, NSW 2122 (AU)
(74) Representative: Vossius & Partner Patentanwälte Rechtsanwälte mbB
(86) International application number: PCT/AU1999/001169
(87) International publication number: WO 2000/038772

(56) References cited:
- WO-A-80/01645
- WO-A-98/26830
- WO-A1-97/33641
- DE-U1- 29 721 766
- GB-A- 2 145 335
- GB-A- 2 219 844
- US-A- 3 419 031
- US-A- 3 518 989
- US-A- 4 088 349
- US-A- 4 648 635
- US-A- 4 870 963
- US-A- 5 163 424
- US-A- 5 330 437
- US-A- 5 398 673
- US-A- 5 438 981
- US-A- 5 645 047
- US-A- 5 662 101

## Description

### FIELD OF THE INVENTION

The present invention relates to an anti-asphyxia valve.

The valve has been developed primarily for use between a patient and a means to deliver a breathable gas to the patient, such as is used in the Continuous Positive Airway Pressure (CPAP) treatment of Obstructive Sleep Apnea (OSA) syndrome, and will be described hereinafter with reference to this application. The valve is also suitable for use in other gas delivery systems, such as those used in assisted respiration and Non-Invasive Positive Pressure Ventilation (NIPPV).

### BACKGROUND OF THE INVENTION

Nasal masks are currently employed for various purposes, including the delivery of oxygen to persons who suffer from lung disease or who are exposed to rareified atmospheres, for administering anaesthetic gases and for delivering pressurised air to persons who suffer from sleep disordered breathing conditions, such as OSA, or for the delivery of non-invasive ventilation. The mask cushions are usually moulded from a relatively soft, resilient, elastic material and they are shaped during manufacture to match the facial contours of an average intended wearer. However, a problem with the known types of masks is that, because individuals vary so much from the average, the masks must be forced against their inherent resiliency to deform and so adapt to the shapes of the users in order to avoid gas leakage. This requires that the masks be secured firmly by retaining straps or harnesses in order to prevent air leakage.

Flow generators are typically utilised to deliver a breathable gas (ie. air) to a patient wearing the mask. In CPAP treatment, gas is delivered to the patient's airways at about 2-30 cm H₂O above atmospheric pressure. The flow generator is generally connected to flexible tubing which is secured to the mask worn by the patient. If the flow generator's operation is interrupted as a result of a power outage or other mechanical or electrical failure, there may be a significant build up of carbon dioxide in the mask as the patient's exhaled air is not washed out of outlet vents which are usually contained in the mask. This may present a health problem to the patient.

There have been numerous patents which have addressed some sort of safety valve for gas or air delivery masks. An example of such a patent is U.S. Patent No. 5,438,981. This patent discloses a counter balanced, rigid valve element which depending on the gas flow, either covers an opening to the ambient air or covers the gas flow airway such that the air or breathing gas is forced out into the ambient air opening. However, this system suffers from being a fairly complicated and expensive system whose correct operation relies on a counter balanced moving part moving relative to its housing. Further, if any condensation from the air gets on or around the balanced valve element, the operation of this valve element can be compromised. This valve is also difficult to clean.

Applicant's international PCT patent application No. PCT/AU97/00849 discloses a valve having a single valve element. However, whilst being simpler than preceding valves of this type, the valve shown in PCT/AU97/00849 still relies on the use of a rigid valve element moving relative to its housing and biased by magnets.

US 3 419 031 A discloses a breathing valve used to control the gas flow during the different phases of breathing. The breathing valve has a resilient valve element in which opposed inlet and outlet conducts determine in mutually opposed spaced annular inlet and outlet seats. The valve is adapted to direct a forced flow of air or gas from a source such as a breathing bag or other artificial breathing machine to the lungs of a patient.

US 5 645 047 A gives information about an inhalation mask with a valve device of the type used for breathing apparatus including a mask section that is adapted for mating with a desired subject, such as about mouth or nose. An inlet tube communicates a desired fluid, such as oxygen, into an interior chamber of the mask. A plate assembly has a thin, pliable outer section to act as a flapper. The plate mount retains the plate assembly, and has at least one pair of passages formed therethrough for communicating the fluid out of the interior chamber and an external fluid into the interior chamber.

US 5 163 424 A shows a disposable resuscitator including an elongated, elastically compressible squeeze bag having a first opening, a one-way valve for the intake of oxygen - containing gas into the bag mounted in the first opening, a second opening which is an airtight communication with the valve housing in the form of a transparent pipe having a pipe stub for the attachment of the resuscitator to a facial mask, and an outlet for exhalation air, the tubular valve housing containing a valve including a valve seat and a disk-shaped valve body of an elastomeric material.

It is an object of the present invention to provide an improved valve of simpler construction than those prior art valves discussed above.

### SUMMARY OF THE INVENTION

The invention is defined by the features of the independent claim.

The operating threshold can be altered to suit particular applications. For example, a valve suitable for use in adult ventilatory assist therapy has an operating threshold of about 2 cm H₂O.

The second portion preferably completely occludes the ports in the closed position.

Preferably, the housing is comprised of two housing parts which are releasably engageable with one another. In an embodiment, the housing parts engage by way of bayonet style fittings.

Desirably, the housing includes a gas inlet in the form of a first substantially frusto-conical portion adapted to frictionally engage a flexible conduit in fluid communication with the means to deliver a breathable gas to the patient and a gas outlet in the form of a second substantially frusto-conical portion adapted to engage a mask or a flexible or rigid conduit in fluid communication with the mask. The frusto-conical portions preferably taper from a smaller distal end to a larger proximal end relative to the housing of the inlet valve.

Desirably also, one of the gas inlet or outlet includes a snap-engageable and detacheable swivel portion adapted to engage the mask or flexible conduit. In a preferred embodiment, the inlet and outlet are respectively provided on one of the two housing parts.

### BRIEF DESCRIPTION OF THE DRAWINGS

Preferred embodiments of the invention will now be described, by way of example only, with reference to the accompanying drawings in which:
Fig. 1 is a general schematic drawing of a system comprising a flow generator being connected to a valve and mask via tubing in which the mask is connected to a patient;
Fig. 2 is a side view of a valve of the present invention;
Fig. 3A is a cross sectional view of Fig. 2 in which the flow generator is not operating;
Fig. 3B is a cross sectional view of Fig. 2 in which the flow generator is operating and generating a pressure differential below the operating threshold;
Fig. 4 is a cross sectional view of the valve of Fig. 2 in which the flow generator is operating and generating a pressure differential above the operating threshold;
Fig. 5 is a perspective view of an alternative embodiment of the present invention wherein the valve is attached to a mask having a CO₂ gas washout vent;
Fig. 6 is a cross sectional view of a further embodiment of the present invention wherein the valve has a unitary housing;
Fig. 7 is a cross sectional view of a yet further embodiment of the present invention wherein the valve includes a swivel conduit connector;
Fig. 8 is a perspective view of another embodiment of the present invention wherein the valve is integral with a mask;
Fig. 9 is a cross sectional view of an embodiment of a flap means;
Fig. 10 is a cross sectional view of another embodiment of a flap means;
Fig. 11 is a cross sectional view of yet another embodiment of a flap means;
Fig. 12 is a perspective view of a further embodiment of a flap means;
Fig. 13 is a perspective view of a further embodiment of a flap means;
Fig. 14 is a perspective view of a further embodiment of a flap means;
Fig. 15 is a perspective view of a further embodiment of a flap means;
Fig. 16 is a perspective view of the flap means shown in Fig. 11;
Fig. 17 is a perspective view of a further embodiment of a flap means;
Fig. 18 is a half-cutaway view of the flap means shown in Fig. 17;
Fig. 19 is a first sectional view of the flap means of Fig. 17 along the line 19, 20-19, 20;
Fig. 20 is a second sectional view of the flap means of Fig. 17 along the line 19, 20-19, 20;
Fig. 21 is a sectional view of the flap means of Fig. 17 along the line 21-21;
Fig. 22 is a perspetive view of a further embodiment of a flap means;
Fig. 23 is a cross sectional side view of the flap means of Fig. 22 in the open position; and
Fig. 24 is a cross sectional side view of the flap means of Fig. 22 in the closed position.

### DETAILED DESCRIPTION OF THE PREFERRED EMBODIMENTS

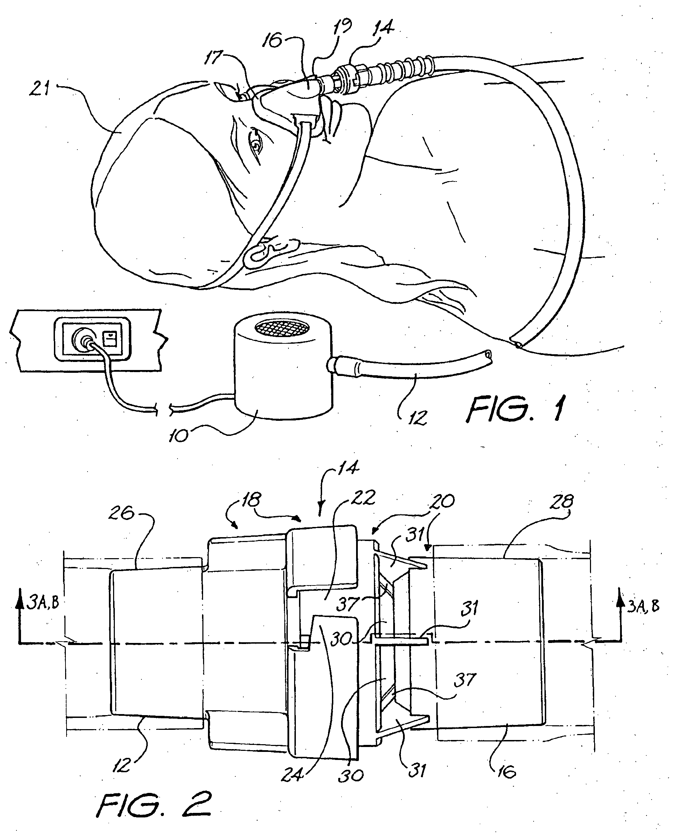

The present invention can be used for any air or oxygen delivery system wherein there is some type of flow generator connected to a tube or airflow conduit which is thereafter secured to a mask which is secured to a patient. The gas being transmitted can be any type of breathable or therapeutic gas. The general schematic of this is shown in Fig. 1 wherein there is a flow generator 10 having a flexible air flow conduit 12 secured to an embodiment of a valve 14 of the present invention which is thereafter connected to a nasal mask 16 of a patient 21. The mask 16 shown includes a mask cushion 17 and a CO₂ gas washout vent 19 and is just one example of numerous types of patient interface.

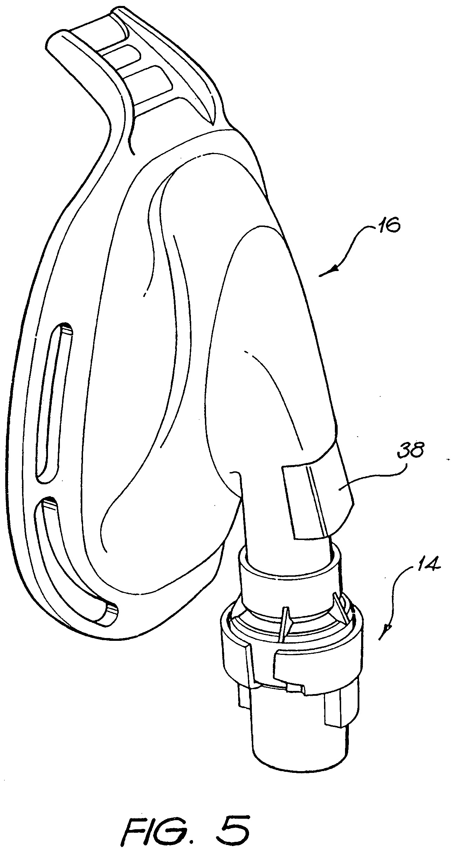

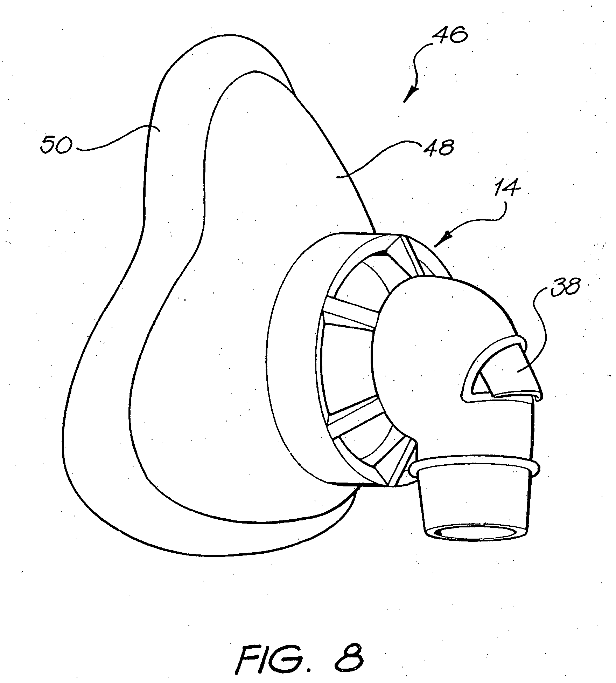

The location of the valve 14 shown in Fig. 1 is just one example of numerous possible locations. The valve 14 could be connected to the mask 16 as shown in Figs. 1 and 5, or it could be an integral part of the mask 16, as shown in Fig. 8. There could also be two or more valves located on a single system. It is preferred to put the valve 14 as close to the mask 16 as possible, or to make it part of the mask 16.

The flow generator 10 produces a flow of breathable gas, typically air, and can be an electric blower, a controlled bottled gas system, a ventilator, or any other type of device that delivers breathable, therapeutic or anaesthetic gas.

The valve 14 shown in Figs. 1 to 4 is comprised of two housing parts 18 and 20 which may be locked together by way of respective male and female bayonet fittings 22 and 24. The housing part 18 includes an inlet in the form of frusto-conical portion 26. The housing part 20 includes an outlet in the form of frusto-conical portion 28. The portions 26 and 28 allow push-on assembly and frictional engagement with the gas supply conduit 12 and the mask housing 16 respectively. The housing part 20 includes six peripherally arranged ports 30 each separated by one of six connecting members 31. A flexible flap means 32 of generally round cross-section is formed from a silicone rubber and has a central orifice 33. The flap means 32 includes a first portion in the form of outer rim 34. The flap means 32 is glued, clamped or otherwise attached or mounted to the second housing part 20 at the outer rim 34. The flap means 32 includes a second portion in the form of flexible flaps 35.

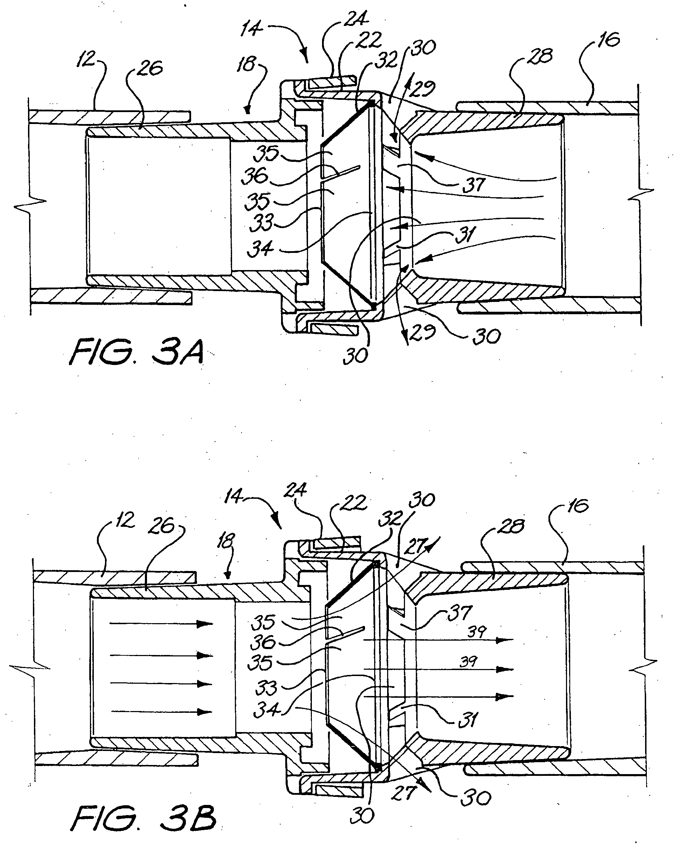

As shown in Fig. 3A, when the difference in the gas pressure between the housing interior and atmosphere is below a predetermined operating threshold of, for example 2 cm H₂O, the flaps 35 are in a relaxed state and inherently biased to an "open" position allowing gas flow from the interior of the housing through the ports 30 and to atmosphere. Accordingly, if the supply of breathable gas falls below the threshold or ceases, the patient 21 is still able to inhale air through the open ports 30 and exhale carbon dioxide out through the open ports 30, as indicated by arrows 29.

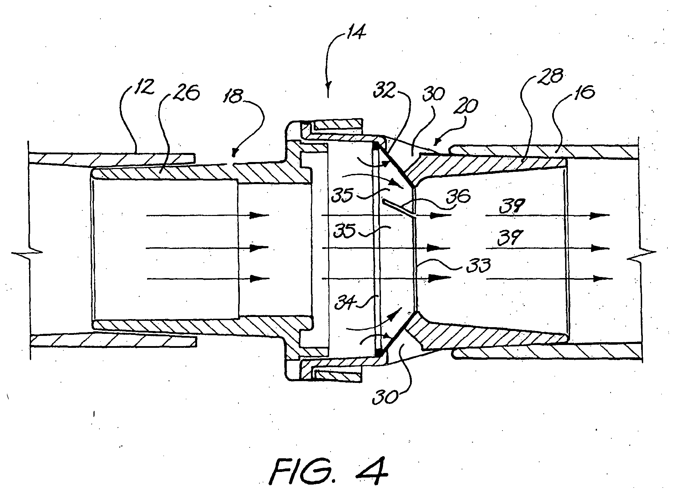

When the breathable gas supply commences or resumes and the difference in the gas pressure between the housing interior and atmosphere builds up to equal or above 2 cm H₂O the flaps 35 move to a "closed" position occluding the ports 30 shown in Fig. 4. Thereafter the flaps 35 are maintained closed by the gas pressure in the housing interior being above the predetermined operating threshold. In this closed position all the gas supplied from the flow generator 10 can pass through the orifice 33 of the flap assembly 32 to be delivered to the mask 16 of the patient 21, as indicated by arrows 39.

In the embodiment shown in Figs. 1 to 4, the flap means 32 has three of the flaps 35 which each subtend an angle of approximately 120°. The three flaps 35 are each separated by slits 36 (only one of three slits 36 shown). The slits 36 allows the flaps 35 to flex between the open and closed positions, as shown in Fig. 3A and Fig. 4 respectively, without crinkling or binding. Every second one of the six connecting members 31 includes a flange 37 which abuts adjacent outer edges (adjacent the slits 36) of each of the flaps 35 in the closed position to assist in sealing the ports 30.

The flap means 32 is preferably manufactured by moulding of a single silicone rubber component in the shape shown in Fig. 3A (ie. the open position). The flaps 35 are preferably 0.15 mm thick. The thickness of the flaps are adjusted to suit their application and, in particular, the pressure of the operating threshold. If too flimsy, the flaps will distend or crumple across the ports 30 and may not move to return from the closed position at the correct pressure. If too stiff, the flaps will not move to the closed position at the correct pressure.

Testing of a prototype of the valve 14 shown in Figs. 1 to 4 was conducted with a flow generator connected to the inlet cylindrical portion 26 via an air flow conduit. A mask was connected to the valve 14 at the outlet cylindrical portion 28. The mask cushion seals the mask interior relative to the wearer's face such that the only gas flow from the mask 16 to atmosphere is through the mask gas washout vent.

With this arrangement the flap assembly 32 closed the ports 30 at an approximately 2 cm H₂O pressure difference (operating threshold) between the interior of the valve 14 and atmosphere.

The inherent resilience of the silicone rubber flaps 35 re-opened the ports 30 when the pressure difference (operating threshold) between the interior of the valve 14 and atmosphere fell below approximately 2 cm H₂O.

Fig. 3B shows the flap assembly 32 in the open position when the flow generator is operating but the pressure difference between the valve interior and atmosphere is below the operating threshold. Under these conditions, some of the supplied gas passes through the ports 30 and the remainder passes through the valve outlet 28 to the mask, as indicated by arrows 27 and 39 respectively.

As the flow through the valve outlet 28 is thus less than the supplied flow through the valve inlet 26, a pressure differential is created between the downstream side of the flaps 35 (that side adjacent the valve outlet 28) and the upstream side of the flaps 35 (that side adjacent the valve inlet 26) which forces the flaps 35 to deform against their inherent resilience towards the ports 30 and, ultimately, to the closed position shown in Fig. 4.

When in the closed position shown in Fig. 4, there is no gas flow through the ports 30. Under these conditions, a pressure differential between the valve interior and atmosphere above the operating threshold will maintain the flaps 35 in the closed position.

The inherent resilience of the flaps 35 moves the flaps 35 away from the ports 30 and towards the open position when the pressure difference between the valve interior and atmosphere falls below the operating threshold.

Fig. 5 illustrates another embodiment in which the valve 14 is attached to another type of mask 16 that includes a CO₂ gas washout vent 38.

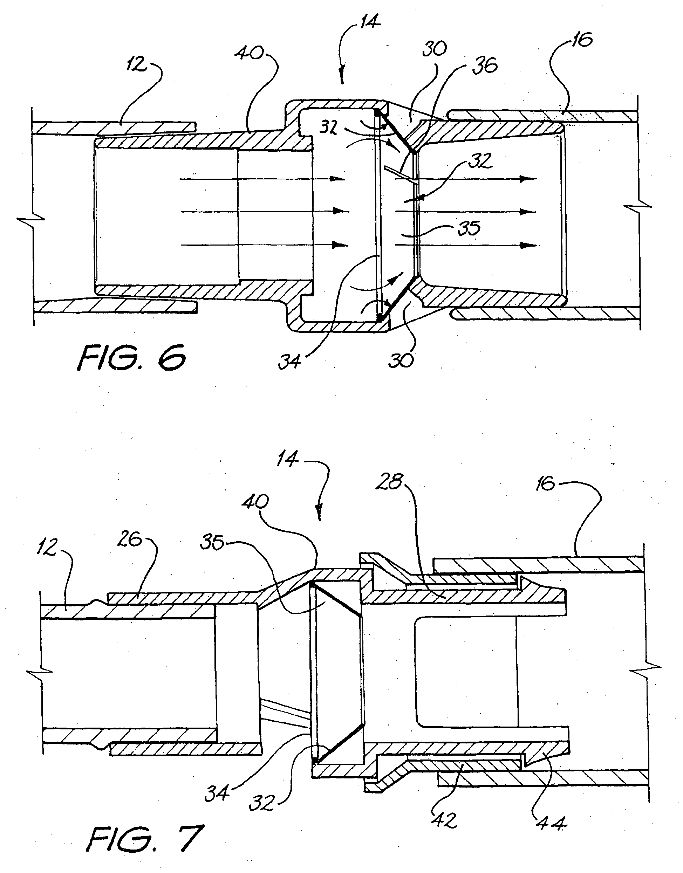

Fig. 6 illustrates an embodiment of the valve 14 having a unitary housing 40.

Fig. 7 illustrates another embodiment of the valve 14 having a unitary housing 40 and a swivel connector 42 that snap-engages with the cylindrical portion 28 over resilient fingers 44. This embodiment obviates the need for a separate swivel connector elsewhere in the gas supply circuit.

In another embodiment (not shown) the swivel connection 42 is used in conjunction with the unitary housing 40.

Fig. 8 illustrates a further embodiment of the valve 14 incorporated into a mask 46 having a mask shell 48 and a mask cushion 50. In this embodiment, the valve 14 is integrally formed with the mask shell 48 thereby obviating the push-on connection between the mask 16 and the valve 14.

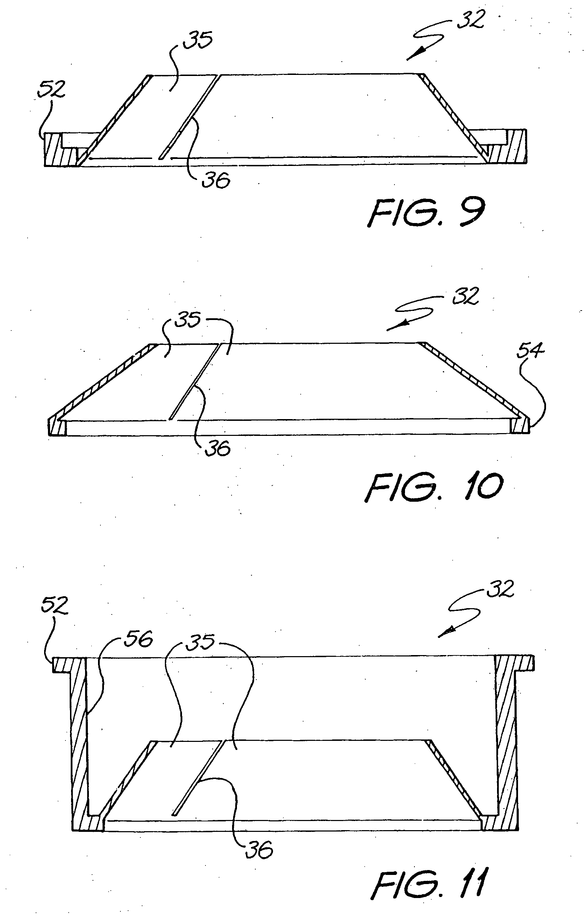

Fig. 9 shows an embodiment of the flap means 32 which includes an external rim 52 of stepped cross section which assists in locating the flap means 32 in the housing(s). The rim 54 is received within a corresponding recess in the housing to facilitate locating and mounting the flap means 32 in the housing.

Fig. 10 shows another embodiment of the flap means 32 having an internal rim 54 of rectangular cross section.

Fig's. 11 and 16 shows yet another embodiment of the flap means 32 having a substantially cylindrical formation 56 between the flaps 35 and the rim 52. The cylindrical formation 56 and the rim 52 facilitate locating the flap means 32 with correct orientation in the housing.

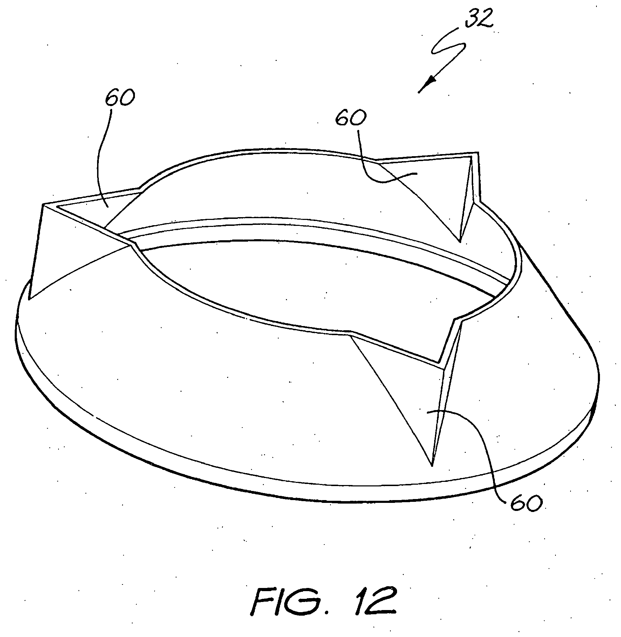

Fig. 12 illustrates a further embodiment of the flap means 32 which includes a series of pleats or folds 60 which flex to allow movement of the flap means 32 between the open and closed positions.

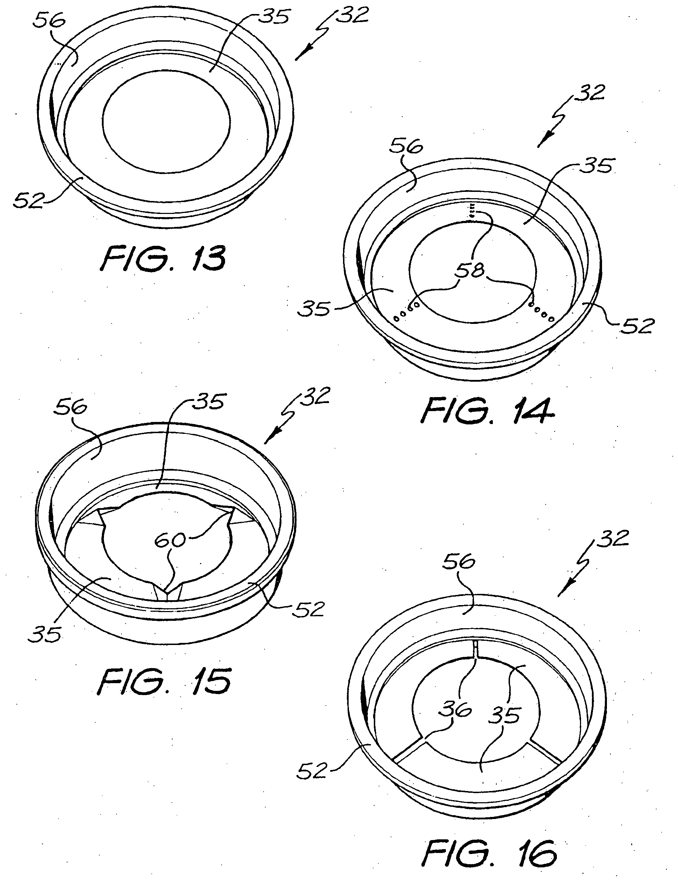

Fig. 13 shows another embodiment of the flap means 32 similar to that shown in Fig. 11 but without the slits 36. This embodiment thus has only a single flap 35 which distorts when moving between the open and closed positions.

Fig. 14 shows an embodiment similar to that of Fig. 13 but including three rows of perforations 58 which provide localised flexability to assist in movement of the flaps 35 between the open and closed positions.

Fig. 15 shows an embodiment of a flap means 32 that has the cylindrical formation 56 of the embodiment of Fig's. 11 and 16 and the pleats or folds 60 of the embodiment of Fig. 12.

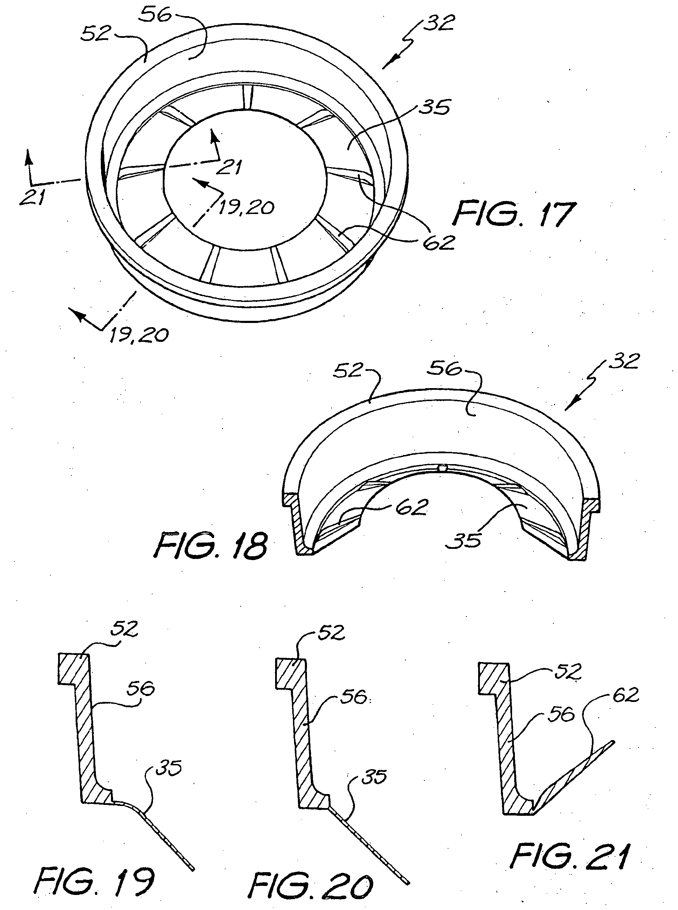

Figs. 17 and 18 show a further embodiment of a flap means 32 with a single flap 35 that includes a number of radial protuberances or ribs 62 of greater thickness than the flap 35. In the embodiment shown, the ribs 62 are of equal thickness. In other embodiments (not shown) the ribs are of unequal thickness and, for example, can be thicker on one side for operation in applications where flow is different across the flap means such as in a curved or angled conduit or the like.

Figs. 19 and 20 show examples of the cross-section the flap 35 can assume in the closed position.

Fig. 21 show an example of the cross-section the ribs 62 can assume in the open position.

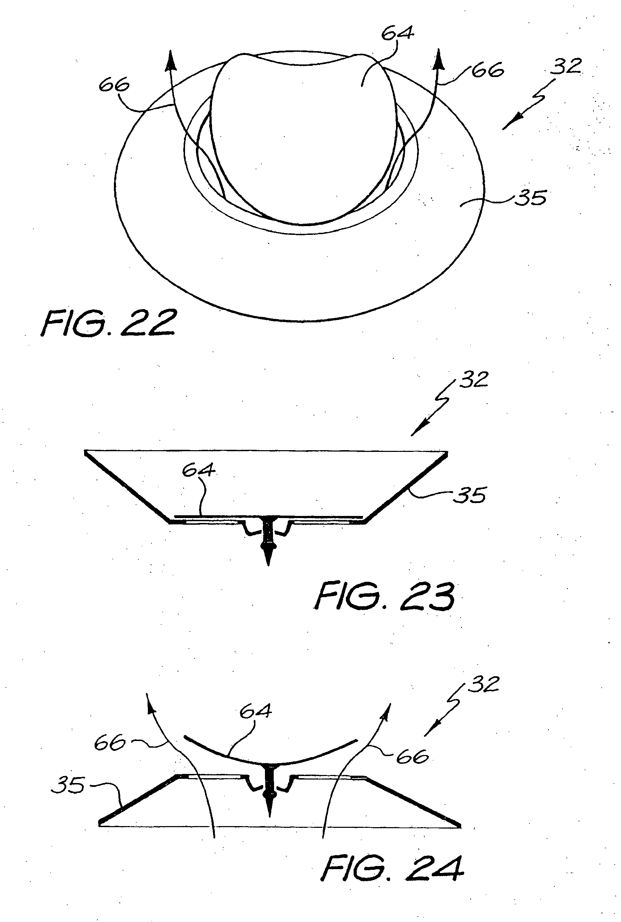

Figs. 22 and 24 are schematic views of another embodiment of a flap means 32 that includes a one-way valve device 64 adapted to only allow gas flow in the direction of the patient. The one-way valve device 64 is known as a non-rebreathe valve.

Figs. 22 and 24 show the flap means 32 in the closed position and the one-way valve 64 in the open position, thereby allowing gas flow to the patient, as indicated by arrows 66. Fig. 23 shows the flap means 32 in the open position and the one-waxy valve 64 in the closed position, thereby directing all of the gas flow directed through the ports of the valve to atmosphere.

Another embodiment of the invention (not shown) includes a port or series of ports that function as both the flap assembly port(s) and the mask CO₂ gas washout port.

In this embodiment, the port(s) and the flap assembly are sized so the port(s) is/are not totally occluded by the flap assembly in the closed position. Accordingly, in the closed position the port(s) is/are occluded to an extent that it/they are of a size suitable to function as the mask CO₂ gas washout vent. When the pressure differential between the interior of the valve and atmosphere is below the operational threshold, the flap assembly moves to the open position and the port(s) to atmosphere is/are enlarged to a size suitable to function as the anti-asphyxia port(s).

The present invention has the advantage of being able to be used with nasal, mouth mask and full face (nose and mouth) mask systems for both adults and infants. In the situation of infants, the airflow is generally less, and thus the force needed to flex the flap assembly into the closed position is lowered accordingly.

The valve according to the present invention can be used for any type of air delivery system, it is preferably used in CPAP applications for the treatment of OSA or Non-Invasive Positive Pressure Ventilation (NIPPV).

Preferred embodiments of the valve of the present invention have the advantage of being able to operate independent of orientation. That is, although the valve has to be connected in the right direction between the flow generator and the mask, it can be inverted, held sideways, etc. which often occurs during the time when the patient sleeps.

Another advantage of the valve of the present invention is it has only one moving or flexing part providing consistent operation.

Further, the valve can be disassembled, cleaned and reassembled very easily at home or at a hospital or clinic due to it having less parts.

The valve of the present invention is also very quiet in operation.

Although the invention has been described with reference to specific examples, it will be appreciated by those skilled in the art that the invention may be embodied in many other forms. In particular, a valve of the present invention may be constructed of components which have dimensions, configurations and mechanical properties (including the mechanical properties of the flap assembly) that vary from those of the prototype. Such valves can have operating thresholds different from the prototype valve which achieved a closure at 2 cm H₂O. The actual dimensions, configurations and mechanical properties will be chosen to achieve a valve having performance characteristic including operating threshold that will meet the specific needs of the chosen application.

## Claims

1. An anti-asphyxia valve adapted to, in use, be disposed between a patient and means to deliver a breathable gas to the patient, the valve (14) comprising a housing (18, 20, 40) having an interior, at least one port to provide fluid communication between the housing interior and atmosphere, the valve (14) being **characterised in that** the valve (14) includes a flap means (32) comprising a first portion (34) adapted for mounting to the housing and a second portion (35) adapted to flex between a first biased open position allowing gas to pass from the housing interior through the port(s) to atmosphere when the difference in gas pressure in the housing interior and atmosphere is below a predetermined operating threshold and a second forced closed position substantially occluding the port(s) when the difference in gas pressure between the housing interior and atmosphere is substantially equal to or above the operating threshold, whereby the valve (14) allows the patient to inhale and exhale gas from and to atmosphere when the means (10) to deliver an operating breathable gas generates less pressure than said threshold gas pressure, wherein the second portion of the flap means (32) terminates in an internal orifice.

2. The valve (14) as claimed in claim 1, wherein the second portion (35) completely occludes the ports in the closed position.

3. The valve (14) as claimed in claim 1 or 2, wherein the housing (18, 20, 40) includes a gas inlet in the form of a first substantially frusto-conical portion (26) adapted to frictionally engage a flexible conduit (12) in fluid communication with the means to deliver a breathable gas to the patient and a gas outlet in the form of a second substantially frusto-conical portion (28) adapted to engage a mask (16) or a flexible or rigid conduit in fluid communication with the mask.

4. The valve (14) as claimed in claim 3, wherein the frusto-conical portions (26, 28) taper from a smaller distal end to a larger proximal end relative to the housing (18, 20, 40) of the inlet valve.

5. The valve (14) as claimed in claim 3 or 4, wherein one of the gas inlet (26) or outlet (28) includes a snap-engageable swivel portion (42) adapted to engage the mask (16) or flexible conduit (12).

6. The valve (14) as claimed in any one of the preceding claims, wherein the housing (18, 20) is comprised of two housing parts (18, 20) which are releasably engageable with one another.

7. The valve (14) as claimed in claim 6, wherein the housing parts (18, 20) engage by way of bayonet style fittings.

8. The valve (14) as claimed in claim 6 or 7, when appended to claim 5, wherein the inlet (26) and outlet (28) are respectively provided on one of the two housing parts (18, 20).

9. The valve (14) as claimed in any one of claims 1 to 5, wherein the housing (40) is of unitary construction.

10. The valve (14) as claimed in any one of the preceding claims, wherein the housing (18, 20, 40) includes a plurality of ports spaced about the periphery thereof and the second portion of the flap means (32) of the flap means includes a like plurality of flaps (35).

11. The valve (14) as claimed in claim 10, wherein the housing (18, 20, 40) includes three pairs of ports (30) and the second portion of the flap means (32) includes three flaps (35) each adapted to close adjacent pairs of the ports (30).

12. The valve (14) as claimed in any one of the claims 1 to 9, wherein the second portion of the flap means (32) is a single flap which (35) is adapted to occlude all the port(s) (30) in the second position.

13. The valve (14) as claimed in claim 12, wherein the single flap (35) includes perforations, ribs, pleats or folds or the like.

14. The valve (14) as claimed in any one of the preceding claims, wherein the first and second portions are integrally formed.

15. The valve (14) as claimed in any one of claims 1 to 13, wherein the first and second portions are initially formed from separate components that are later attached to each other.

16. The valve (14) as claimed in any one of the preceding claims, wherein the first portion includes a rim (52) adapted to assist in mounting the flap means (32) to the housing.

17. The valve (14) as claimed in claim 16, wherein the rim is an external rim of rectangular cross section which is adapted to engage an internal recess of substantially like cross-section in the housing.

18. The valve (14) as claimed in claim 16 or 17, wherein the first portion includes a substantially cylindrical portion between the rim and the second portion.

19. The valve (14) as claimed in claim 18, wherein the rim and/or the substantially cylindrical portion are tapered.

20. The valve (14) as claimed in claim 1, wherein the orifice includes a one-way valve adapted to only allow gas flow through the orifice in a direction towards the patient.

21. The valve (14) as claimed in any one of the preceding claims, wherein the flap means (32) is substantially round in cross-section.

22. The valve (14) as claimed in any one of claims 1 to 20, wherein the cross section of the flap means (32) is full or part elliptical or rectangular or other out-of-round shapes.

23. The valve (14) as claimed in any one of the preceding claims, wherein the housing (18, 20, 40) is manufactured from plastics material.

24. The valve (14) as claimed in claim 23, wherein the plastics material is polycarbonate.

25. The valve (14) as claimed in any one of the preceding claims wherein the flap assembly (32) is preferably manufactured from a flexible elastomeric material.

26. The valve (14) as claimed in claim 25, wherein the elastomeric material is a silicone rubber.

27. The valve (14) as claimed in any one of the preceding claims wherein the valve (14) is integral with a mask (16).

28. The valve (14) as claimed in any one of the preceding claims, wherein the operating threshold is about 2cm H₂O.

## Patentansprüche

1. Anti-Asphyxie-Ventil, das eingerichtet ist, im Gebrauch zwischen einem Patienten und einem Mittel zur Abgabe eines Atemgases an den Patienten angeordnet zu werden, wobei das Ventil (14) ein Gehäuse (18, 20, 40) aufweist, das ein Inneres aufweist, wobei mindestens eine Öffnung eine Fluidverbindung zwischen dem Gehäuseinneren und der Atmosphäre bereitstellen soll, wobei das Ventil (14) **dadurch gekennzeichnet ist, dass** das Ventil (14) ein Klappenmittel (32) aufweist, das einen ersten Abschnitt (34), der zum Anbringen am Gehäuse eingerichtet ist, und einen zweiten Abschnitt (35) aufweist, der eingerichtet ist, sich zwischen einer ersten vorgespannten offenen Position, die es zulässt, dass Gas aus dem Gehäuseinneren durch die Öffnung(en) zur Atmosphäre geht, wenn die Differenz des Gasdrucks im Gehäuseinneren und der Atmosphäre unter einer vorgegebenen Ansprechschwelle liegt, und einer zweiten erzwungenen geschlossenen Position zu biegen, die im Wesentlichen die Öffnung(en) verschließt, wenn die Differenz des Gasdrucks zwischen dem Gehäuseinneren und der Atmosphäre im Wesentlichen gleich der Ansprechschwelle ist oder darüber liegt, wodurch es das Ventil (14) dem Patienten ermöglicht, Gas aus der und zur Atmosphäre ein- und auszuatmen, wenn das Mittel (10) zum Abgeben eines Betriebsatemgases weniger Druck als den Schwellengasdruck erzeugt, wobei der zweite Abschnitt des Klappenmittels (32) in einer inneren Mündung endet.

2. Ventil (14) nach Anspruch 1, wobei der zweite Abschnitt (35) in der geschlossenen Position die Öffnungen vollständig verschließt.

3. Ventil (14) nach Anspruch 1 oder 2, wobei das Gehäuse (18, 20, 40) einen Gaseinlass in der Form eines ersten, im Wesentlichen kegelstumpfförmigen Abschnitts (26), der eingerichtet ist, mit einer flexiblen Leitung (12) in Reibungseingriff zu treten, die mit dem Mittel zum Abgeben eines Atemgases an den Patienten in Fluidverbindung steht, und einen Gasauslass in der Form eines zweiten im Wesentlichen kegelstumpfförmigen Abschnitts (28) aufweist, der eingerichtet ist, mit einer Maske (16) oder einer flexiblen oder starren Leitung in Eingriff zu treten, die mit der Maske in Fluidverbindung steht.

4. Ventil (14) nach Anspruch 3, wobei sich die kegelstumpfförmigen Abschnitte (26, 28) relativ zum Gehäuse (18, 20, 40) des Einlassventils von einem kleineren distalen Ende zu einem größeren proximalen Ende verjüngen.

5. Ventil (14) nach Anspruch 3 oder 4, wobei der Gaseinlass (26) oder -Auslass (28) einen in Schnappeingriff bringbaren Schwenkabschnitt (42) aufweist, der eingerichtet ist, mit der Maske (16) oder der flexiblen Leitung (12) in Eingriff zu treten.

6. Ventil (14) nach einem der vorhergehenden Ansprüche, wobei das Gehäuse (18, 20) aus zwei Gehäuseteilen (18, 20) besteht, die lösbar miteinander in Eingriff gebracht werden können.

7. Ventil (14) nach Anspruch 6, wobei die Gehäuseteile (18, 20) mittels einer Bajonettbefestigung in Eingriff treten.

8. Ventil (14) nach Anspruch 6 oder 7, wenn von Anspruch 5 abhängig, wobei der Einlass (26) und Auslass (28) jeweils an einem der beiden Gehäuseteile (18, 20) vorgesehen ist.

9. Ventil (14) nach einem der Ansprüche 1 bis 5, wobei das Gehäuse (40) aus einem einheitlichen Aufbau besteht.

10. Ventil (14) nach einem der vorhergehenden Ansprüche, wobei das Gehäuse (18, 20, 40) eine Vielzahl von Öffnungen aufweist, die um dessen Umfang beabstandet sind, und der zweite Abschnitt des Klappenmittels (32) eine gleiche Vielzahl von Klappen (35) aufweist.

11. Ventil (14) nach Anspruch 10, wobei das Gehäuse (18, 20, 40) drei Paare von Öffnungen (30) aufweist und der zweite Abschnitt des Klappenmittels (32) drei Klappen (35) aufweist, die eingerichtet sind, benachbarte Paare der Öffnungen (30) zu verschließen.

12. Ventil (14) nach einem der Ansprüche 1 bis 9, wobei der zweite Abschnitt des Klappenmittels (32) eine einzelne Klappe (35) ist, die eingerichtet ist, alle Öffnung(en) (30) in der zweiten Position zu verschließen.

13. Ventil (14) nach Anspruch 12, wobei die einzelne Klappe (35) Perforationen, Rippen, Sicken oder Falten oder dergleichen aufweist.

14. Ventil (14) nach einem der vorhergehenden Ansprüche, wobei der erste und zweite Abschnitt integral ausgebildet sind.

15. Ventil (14) nach einem der Ansprüche 1 bis 13, wobei der erste und zweite Abschnitt anfänglich aus getrennten Komponenten ausgebildet sind, die später aneinander befestigt werden.

16. Ventil (14) nach einem der vorhergehenden Ansprüche, wobei der erste Abschnitt einen Rand (52) aufweist, der eingerichtet ist, das Anbringen des Klappenmittels (32) am Gehäuse zu unterstützen.

17. Ventil (14) nach Anspruch 16, wobei der Rand ein äußerer Rand mit einem rechteckigen Querschnitt ist, der eingerichtet ist, mit einer inneren Aussparung mit einem im Wesentlichen gleichen Querschnitt im Gehäuse in Eingriff zu treten.

18. Ventil (14) nach Anspruch 16 oder 17, wobei der erste Abschnitt einen im Wesentlichen zylindrischen Abschnitt zwischen dem Rand und dem zweiten Abschnitt aufweist.

19. Ventil (14) nach Anspruch 18, wobei der Rand und/oder der im Wesentlichen zylindrische Abschnitt verjüngt sind.

20. Ventil (14) nach Anspruch 1, wobei die Mündung ein Einwegventil aufweist, das eingerichtet ist, nur in eine Richtung einen Gasfluss durch die Mündung zum Patienten zuzulassen.

21. Ventil (14) nach einem der vorhergehenden Ansprüche, wobei das Klappenmittel (32) einen im Wesentlichen runden Querschnitt aufweist.

22. Ventil (14) nach einem der Ansprüche 1 bis 20, wobei der Querschnitt des Klappenmittels (32) vollständig oder teilweise elliptische oder rechteckige oder andere unrunde Formen aufweist.

23. Ventil (14) nach einem der vorhergehenden Ansprüche, wobei das Gehäuse (18, 20, 40) aus einem Kunststoff hergestellt ist.

24. Ventil (14) nach Anspruch 23, wobei der Kunststoff Polykarbonat ist.

25. Ventil (14) nach einem der vorhergehenden Ansprüche, wobei die Klappenmittel (32) vorzugsweise aus einem flexiblen Elastomermaterial hergestellt ist.

26. Ventil (14) nach Anspruch 25, wobei das Elastomermaterial Silikongummi ist.

27. Ventil (14) nach einem der vorhergehenden Ansprüche, wobei das Ventil (14) mit einer Maske (16) integral ist.

28. Ventil (14) nach einem der vorhergehenden Ansprüche, wobei die Ansprechschwelle etwa 2 cm H₂O beträgt.

## Revendications

1. Valve anti-asphyxie prévue pour être, en cours d'utilisation, disposée entre un patient et un moyen d'administration d'un gaz respirable au patient, ladite valve (14) comprenant un boîtier (18, 20, 40) présentant un compartiment intérieur, au moins un orifice permettant une communication fluidique entre l'intérieur du boîtier et l'atmosphère, la valve (14) étant **caractérisée en ce que** la valve (14) inclut une unité de clapet (32) comprenant une première partie (34) prévue pour être montée sur le boîtier et une deuxième partie (35) prévue pour pivoter entre une première position d'ouverture contrainte permettant le passage de gaz de l'intérieur du boîtier vers l'atmosphère par l'orifice ou les orifices si la différence de pression gazeuse entre l'intérieur du boîtier et l'atmosphère est inférieure à un seuil de fonctionnement prédéfini, et une deuxième position de fermeture forcée obturant sensiblement l'orifice ou les orifices si la différence de pression gazeuse entre l'intérieur du boîtier et l'atmosphère est sensiblement égale ou supérieur au seuil de fonctionnement, ladite valve (14) permettant au patient d'inspirer du gaz de l'atmosphère et d'expirer du gaz vers l'atmosphère, si le moyen (10) d'administration d'un gaz respirable opératoire génère une pression inférieure au seuil de pression gazeuse, où la deuxième partie de l'unité de clapet (32) se termine dans un orifice interne.

2. Valve (14) selon la revendication 1, où la deuxième partie (35) obture complètement les orifices en position de fermeture.

3. Valve (14) selon la revendication 1 ou la revendication 2, où le boîtier (18, 20, 40) comporte une entrée de gaz sous la forme d'une première partie (26) sensiblement tronconique prévue pour raccorder par friction un tuyau flexible (12) en communication fluidique avec le moyen d'administration d'un gaz respirable au patient et un sortie de gaz sous la forme d'une deuxième partie (28) sensiblement tronconique prévue pour raccorder un masque (16), ou un tuyau flexible ou rigide en communication fluidique avec ledit masque.

4. Valve (14) selon la revendication 3, où les parties tronconiques (26, 28) s'amincissent entre une extrémité étroite distale et une extrémité large proximale par rapport au boîtier (18, 20, 40) de la valve d'admission.

5. Valve (14) selon la revendication 3 ou la revendication 4, où l'entrée (26) ou la sortie (28) de gaz comporte une partie pivotante enclenchable (42) prévue pour le raccordement du masque (16) ou du tuyau flexible (12).

6. Valve (14) selon l'une des revendications précédentes, où le boîtier (18, 20) est constitué de deux pièces (18, 20) de boîtier raccordables l'une à l'autre de manière amovible.

7. Valve (14) selon la revendication 6, où les pièces (18, 20) de boîtier sont raccordées au moyen de connexions à baïonnette.

8. Valve (14) selon la revendication 6 ou la revendication 7, si dépendantes de la revendication 5, où l'entrée (26) et la sortie (28) sont respectivement prévues sur une des deux pièces (18, 20) de boîtier.

9. Valve (14) selon l'une des revendications 1 à 5, où le boîtier (40) est construit d'un seul tenant.

10. Valve (14) selon l'une des revendications précédentes, où le boîtier (18, 20, 40) présente une pluralité d'orifices espacés sur sa périphérie, et où la deuxième partie de l'unité de clapet (32) présente une pluralité correspondante de clapets (35).

11. Valve (14) selon la revendication 10, où le boîtier (18, 20, 40) présente trois paires d'orifices (30) et où la deuxième partie de l'unité de clapet (32) présente trois volets (35) prévus pour obturer des paires d'orifices (30) adjacentes.

12. Valve (14) selon l'une des revendications 1 à 9, où la deuxième partie de l'unité de clapet (32) est un seul volet (35) prévu pour obturer l'ensemble des orifices (30) en deuxième position.

13. Valve (14) selon la revendication 12, où le seul volet (35) présente des perforations, des nervures, des plis, des rabats ou similaires.

14. Valve (14) selon l'une des revendications précédentes, où la première et la deuxième parties sont formées d'un seul tenant.

15. Valve (14) selon l'une des revendications 1 à 13, où la première et la deuxième parties sont initialement constituées de composants séparés, raccordés dans un deuxième temps.

16. Valve (14) selon l'une des revendications précédentes, où la première partie présente un rebord (52) prévu pour faciliter le montage de l'unité de clapet (32) sur le boîtier.

17. Valve (14) selon la revendication 16, où le rebord est un rebord extérieur à section transversale rectangulaire, prévu pour s'enclencher dans un évidement intérieur de section transversale sensiblement identique du boîtier.

18. Valve (14) selon la revendication 16 ou la revendication 17, où la première partie présente une section sensiblement cylindrique entre le rebord et la deuxième partie.

19. Valve (14) selon la revendication 18, où le rebord et/ou la section sensiblement cylindrique sont de forme tronconique.

20. Valve (14) selon la revendication 1, où l'orifice comprend une valve unidirectionnelle prévue pour ne permettre le passage de gaz par l'orifice que dans la direction vers le patient.

21. Valve (14) selon l'une des revendications précédentes, où l'unité de clapet (32) est de section transversale sensiblement circulaire.

22. Valve (14) selon l'une des revendications 1 à 20, où la section transversale de l'unité de clapet présente une forme entièrement ou partiellement elliptique ou rectangulaire ou une autre forme non circulaire.

23. Valve (14) selon l'une des revendications précédentes, où le boîtier (18, 20, 40) est fabriqué dans une matière plastique.

24. Valve (14) selon la revendication 23, où la matière plastique est le polycarbonate.

25. Valve (14) selon l'une des revendications précédentes, où l'unité de clapet (32) est préférentiellement fabriquée dans un matériau élastomère souple.

26. Valve (14) selon la revendication 25, où le matériau élastomère est un caoutchouc silicone.

27. Valve (14) selon l'une des revendications précédentes, où ladite valve (14) est formée d'un seul tenant avec un masque (16).

28. Valve (14) selon l'une des revendications précédentes, où le seuil de fonctionnement est d'environ 2 cm de H₂O.
